# EUROPEAN PATENT APPLICATION

(11) **EP 4 286 427 A1**
(43) Date of publication of application: **06.12.2023**
(21) Application number: 22745619.1
(22) Date of filing: 14.01.2022
(51) Int. Cl.: C08F 20/36, C08K 5/13, C08K 5/524, C08L 75/16, C08G 18/08, C08G 18/10, C08G 18/22, C08G 18/38, C08G 18/67, C08G 18/76, A61K 6/30, A61K 6/60, A61K 6/831, A61K 6/887, A61K 6/889, A61K 6/90

(54) **MONOMER COMPOSITION, CURABLE COMPOSITION, MOLDED BODY AND METHOD FOR PRODUCING MONOMER COMPOSITION**

(30) Priority: 26.01.2021 JP 2021010402
(71) Applicant: Mitsui Chemicals, Inc., Tokyo 104-0028 (JP)
(72) Inventor: KAKINUMA, Naoyuki, Sodegaura-shi, Chiba 299-0265 (JP)
(74) Representative: Hoffmann Eitle
(86) International application number: PCT/JP2022/001235
(87) International publication number: WO 2022/163396

(57) **Abstract**

A monomer composition contains: a tin-free catalyst (A); and a (meth)acrylate monomer (D) that is a reaction product of an iso(thio)cyanate compound (B) and an alcohol compound (C). In this monomer composition, a content of an amide compound containing at least one of a carboxylic acid amide group or a sulfonamide group is less than 1 ppm by mass with respect to a total amount of the monomer composition.

## Description

### Technical Field

The present disclosure relates to: a monomer composition; a curable composition; a molded body; and a method of producing a monomer composition.

### Background Art

Urethane (meth)acrylates are known as monomers contained in curable compositions. Urethane (meth)acrylates are (meth)acrylate compounds containing a urethane bond.

Conventionally, a urethane (meth)acrylate is synthesized using a Sn (tin)-based catalyst.

For example, Patent Document 1 discloses a dental composite filler which is a mixture of a ultrafine inactive inert inorganic filler powder and a reaction product of an organic diisocyanate and an oxyalkyl acrylate or an oxyalkyl methacrylate. In the examples of this Patent Document 1, diurethane dimethacrylate is formed by reacting oxypropyl methacrylate and 2,2,4-trimethylhexamethylene diisocyanate using dibutyl tin dilaurate as a catalyst.

Patent Document 1: Japanese Patent Application Laid-Open (JP-A) No. S51-36960

### SUMMARY OF THE INVENTION

### Technical Problem

In recent years, from the standpoint of, for example, reducing the use of a heavy metal, there is a demand for a catalyst alternative to a Sn-based catalyst (e.g., dibutyl tin dilaurate (DBTDL)) as a catalyst to be used in the formation of a urethane (meth)acrylate.

In the case of using a monomer composition containing a specific urethane (meth)acrylate monomer as a raw material of a dental material, the hue of the monomer composition is desired to be suitable for the dental material. The specific urethane (meth)acrylate monomer can be obtained by reacting iso(thio)cyanate compound containing an iso(thio)cyanate group with (meth)acrylate compound containing a hydroxy group using a catalyst. Specifically, it is desired that the hue of the monomer composition does not have a yellow tinge.

An object of one aspect of the disclosure is to provide: a monomer composition which has a hue suitable for a dental material even with the use of a catalyst alternative to a Sn-based catalyst; a curable composition; a molded body; and a method of producing a monomer composition.

### Solution to Problem

Means for solving the above-described problems include the following embodiments.
<1> A monomer composition, containing: a tin-free catalyst (A); and a (meth)acrylate monomer (D) that is a reaction product of an iso(thio)cyanate compound (B) and an alcohol compound (C),
   wherein a content of an amide compound containing at least one of a carboxylic acid amide group or a sulfonamide group is less than 1 ppm by mass with respect to a total amount of the monomer composition.
<2> The monomer composition according to <1>, wherein
   the tin-free catalyst (A) contains a ligand, and
   the ligand contains a group having at least one of a diketone structure or a double bond other than a carbonyl bond.
<3> The monomer composition according to <1> or <2>, wherein the tin-free catalyst (A) contains at least one of a compound represented by the following Formula (X), a compound represented by the following Formula (Y), or a compound represented by the following Formula (Z): 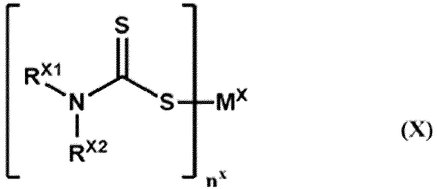 wherein, in Formula (X), each of R^{X1} and R^{X2} independently represents a monovalent hydrocarbon group; M^{X} represents a metal atom other than tin; n^{X} represents a valence of M^{X}; and plural R^{X1}s and R^{X2}s may be the same or different: 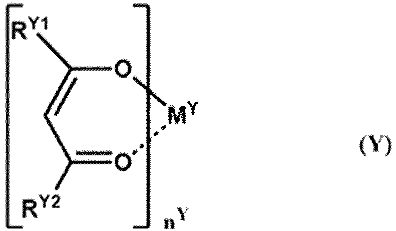 wherein, in Formula (Y), each of R^{Y1} and R^{Y2} independently represents a monovalent hydrocarbon group; M^{Y} represents a metal atom other than tin; n^{Y} represents a valence of M^{Y}; and plural R^{Y1}s and R^{Y2}s may be the same or different: 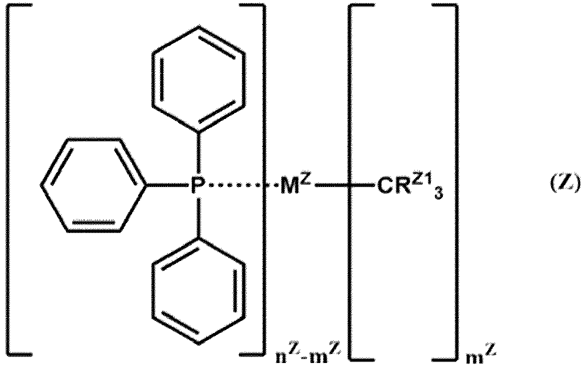 wherein, in Formula (Z), R^{Z1} represents a halogen atom; M^{Z} represents a metal atom other than tin; n^{Z} represents a valence of M^{Z}; and m^{Z} represents 1 or 0.
<4> The monomer composition according to any one of <1> to <3>, wherein the tin-free catalyst (A) contains a metal atom other than tin, and
   the metal atom other than tin is an atom of a metal element (excluding a tin atom) that belongs to at least one of Periods 4 to 6 and at least one of Groups 4 to 14.
<5> The monomer composition according to any one of <1> to <4>, wherein the tin-free catalyst (A) contains at least one selected from the group consisting of a zinc-based catalyst, a copper-based catalyst, an indium-based catalyst, and a platinum-based catalyst.
<6> The monomer composition according to any one of <1> to <5>, wherein the iso(thio)cyanate compound (B) contains an aromatic ring.
<7> The monomer composition according to any one of <1> to <6>, wherein the iso(thio)cyanate compound (B) contains an iso(thio)cyanate compound (b1) not containing a thiourethane bond or, a reaction product (X) of a thiol compound (F) containing two or more mercapto groups and an iso(thio)cyanate compound (b2) not containing a thiourethane bond.
<8> The monomer composition according to <7>, wherein the iso(thio)cyanate compound (b1) and the iso(thio)cyanate compound (b2) each contain at least one selected from the group consisting of m-xylylene diisocyanate, 1,3-tetramethylxylylene diisocyanate, tolylene diisocyanate, phenylene diisocyanate, and 4,4'-diphenylmethane diisocyanate.
<9> The monomer composition according to any one of <1> to <8>, wherein the alcohol compound (C) contains at least one selected from the group consisting of 2-hydroxyethyl (meth)acrylate, 2-hydroxypropyl (meth)acrylate, 2-hydroxybutyl (meth)acrylate, 2-hydroxy-3-phenoxypropyl (meth)acrylate, 4-hydroxybutyl (meth)acrylate, 1,4-cyclohexane dimethanol mono(meth)acrylate, ethylene glycol, and glycerin.
<10> The monomer composition according to any one of <1> to <9>, containing a stabilizer (E) which is at least one of a phosphite compound or a phenolic compound.
<11> The monomer composition according to any one of <1> to <10>, wherein the iso(thio)cyanate compound (B) is a reaction product of the iso(thio)cyanate compound (b2) not containing a thiourethane bond and the thiol compound (F) containing two or more mercapto groups.
<12> A curable composition, containing the monomer composition according to any one of <1> to <11>.
<13> The curable composition according to <12>, further containing a polymerization initiator.
<14> The curable composition according to <12> or <13>, which is a composition for a dental material.
<15> A molded body, comprising a cured product of the curable composition according to any one of <12> to <14>.
<16> A method of producing a monomer composition containing a tin-free catalyst (A) and a (meth)acrylate monomer (D) that is a reaction product of an iso(thio)cyanate compound (B) and an alcohol compound (C), the method comprising:
   mixing the tin-free catalyst (A), the iso(thio)cyanate compound (B), and the alcohol compound (C),
   wherein a content of an amide compound containing at least one of a carboxylic acid amide group or a sulfonamide group is less than 1 ppm by mass with respect to a total amount of the monomer composition.

### Advantageous Effects of Invention

According to one aspect of the disclosure, the following are provided: a monomer composition which has a hue suitable for a dental material even with the use of a catalyst alternative to a Sn-based catalyst; a curable composition; a molded body; and a method of producing a monomer composition.

### DESCRIPTION OF EMBODIMENTS

In the disclosure, those numerical ranges that are expressed with "to" each denote a range that includes the numerical values stated before and after "to" as the lower limit value and the upper limit value, respectively.

In the disclosure, the term "step" encompasses not only a discrete step but also a step that cannot be clearly distinguished from other steps, as long as the intended purpose of the step is achieved.

In the disclosure, when there are plural substances that correspond to a component of a composition, the indicated amount of the component in the composition means, unless otherwise specified, a total amount of the plural substances existing in the composition.

In a set of numerical ranges that are stated in a stepwise manner in the disclosure, the upper limit value or the lower limit value of one numerical range may be replaced with the upper limit value or the lower limit value of other numerical range. Further, in a numerical range stated in the disclosure, the upper limit value or the lower limit value of the numerical range may be replaced with a relevant value indicated in any of Examples.

In the disclosure, "light" is a concept that encompasses active energy rays such as ultraviolet rays and visible light beams.

In the disclosure, "(meth)acrylic monomer" means an acrylic monomer or a methacrylic monomer; "(meth)acryloyl group" means an acryloyl group or a methacryloyl group; "(meth)acrylate" means acrylate or methacrylate; "(meth)acrylic acid" means acrylic acid or methacrylic acid; and "(meth)acrylonitrile" means acrylonitrile or methacrylonitrile.

In the disclosure, "iso(thio)cyanate group" means an isocyanate group or an isothiocyanate group, and "iso(thio)cyanate compound" means an isocyanate compound or an isothiocyanate compound.

In the disclosure, "amide compound" means an amide compound containing at least one of a carboxylic acid amide group or a sulfonamide group.

In the disclosure, the expression "content of an amide compound is less than 1 ppm by mass with respect to a total amount of a monomer composition" encompasses a case where the content of the amide compound is 0 part by mass with respect to the total amount of the monomer composition.

In the disclosure, a -NHC(=O)O- bond and a -NHC(=S)O- bond are collectively referred to as "urethane bond", and a compound containing a (meth)acryloyl group and at least one of a -NHC(=O)O- bond or a -NHC(=S)O- bond is also referred to as "urethane (meth)acrylate".

Preferred constitutions that are described for the monomer composition, the curable composition, the molded body, and the method of producing a monomer composition according to the disclosure may be combined with each other as appropriate and, for example, a constitution disclosed for the monomer composition may be combined with the method of producing a monomer composition or the like as appropriate.

### [Monomer Composition]

The monomer composition of the disclosure is a monomer composition containing: a tin-free catalyst (A); and a (meth)acrylate monomer (D) that is a reaction product of an iso(thio)cyanate compound (B) and an alcohol compound (C) and, in this monomer composition, a content of an amide compound containing at least one of a carboxylic acid amide group (-C(=O)NH₂) or a sulfonamide group (-S(=O)₂NH₂) is less than 1 ppm by mass with respect to a total amount of the monomer composition.

Since the monomer composition of the disclosure has the above-described constitution, it has a hue suitable for a dental material even when a catalyst alternative to a Sn-based catalyst is used therein. Specifically, the hue of the monomer composition of the disclosure does not have a yellow tinge. Therefore, by using the monomer composition of the disclosure, a cured product having a hue suitable for a dental material can be produced.

As described above, from the standpoint of, for example, reducing the use of a heavy metal, there is a demand for a catalyst alternative to a Sn-based catalyst as a catalyst to be used in the formation of a urethane (meth)acrylate. In addition, as a raw material of a dental material, a monomer composition is desired to have a hue suitable for the dental material (e.g., without a yellow tinge).

Meanwhile, when a commercially available product is used as a raw material of an iso(thio)cyanate compound in the presence of a tin-free catalyst (e.g., a zinc-based catalyst), the hue of the resulting monomer composition has a yellow tinge in some cases. Usually, a commercially available iso(thio)cyanate compound contains a stabilizer.

The present inventor intensively studied to newly discover that, in the use of a tin-free catalyst (e.g., a zinc-based catalyst), the main cause of the resulting monomer composition to have a hue with a yellow tinge resides in an amide compound, thereby completing the disclosure. The amide compound is contained as a stabilizer in a commercially available iso(thio)cyanate compound.

As described above, an amide compound may be contained in a commercially available product. For example, by using a purified product obtained by purifying such a commercially available product by a known method as a raw material of a monomer composition, the content of the amide compound can be controlled to be in the above-described range.

Components that may be contained in the monomer composition of the disclosure will now be described.

### <Tin-Free Catalyst (A)>

The monomer composition of the disclosure contains a tin-free catalyst (A).

The tin-free catalyst (A) is a catalyst that contains a metal atom other than a tin atom, such as a zinc atom, a titanium atom, a zirconium atom, a bismuth atom, an iron atom, a copper atom, or a cobalt atom.

The tin-free catalyst (A) is a catalyst used for the generation of a (meth)acrylate monomer (D) (i.e. a reaction of an iso(thio)cyanate compound (B) and an alcohol compound (C)).

In the monomer composition of the disclosure, the tin-free catalyst (A) may be contained singly, or in combination of two or more kinds thereof.

When the tin-free catalyst (A) is a ligand-containing specific compound, the use of the monomer composition as a dental material is unlikely to cause a problem in the hue (e.g., unlikely to generate a yellow tinge). The ligand of the specific compound is a single ligand that is at least one selected from the group consisting of an alkyl group, a carboxyl group, an alkoxy group, and a halogeno group.

On the other hand, when the tin-free catalyst (A) contains a ligand other than the above-described ones, the use of the monomer composition as a dental material may cause a problem in the hue (e.g., generation of a yellow tinge).

However, in the monomer composition of the disclosure, the content of an amide compound is less than 1 ppm by mass. Therefore, even when the tin-free catalyst (A) is a compound other than the above-described specific compound, it is unlikely to cause deterioration of the hue, so that the monomer composition can be provided with a hue that is more suitable for a dental material.

In this manner, from the standpoint of inhibiting deterioration of the hue, the tin-free catalyst (A) according to the disclosure, when it contains a ligand, is preferably the below-described first compound, more preferably the below-described second compound, still more preferably the below-described third compound.

The first compound is a compound other than one in which the ligand is a single ligand that is at least one selected from the group consisting of an alkyl group and a carboxyl group.

The second compound is a compound other than one in which the ligand is a single ligand that is at least one selected from the group consisting of an alkyl group, a carboxyl group, and an alkoxy group.

The third compound is a compound other than one in which the ligand is a single ligand that is at least one selected from the group consisting of an alkyl group, a carboxyl group, an alkoxy group, and a halogeno group.

Examples of the alkyl group that is a ligand include alkyl groups having from 1 to 20 carbon atoms (which may be linear or branched).

Examples of the carboxyl group that is a ligand include carboxyl groups represented by a general formula: R-C(=O)-O-, wherein R represents an alkyl group having from 1 to 20 carbon atoms (which may be linear or branched).

Examples of the alkoxy group that is a ligand include alkoxy groups represented by a general formula: R-O-, wherein R represents an alkyl group having from 1 to 20 carbon atoms (which may be linear or branched).

Examples of the halogeno group that is a ligand include a fluoro group (F-), a chloro group (Cl-), a bromo group (Br-), and an iodine group (I-).

When the tin-free catalyst (A) has an absorbance of 0.05 or more in a wavelength range of from 250 nm to 800 nm, the use of the monomer composition as a dental material may cause a problem in the hue (e.g., generation of a yellow tinge).

However, in the monomer composition of the disclosure, since the content of an amide compound is less than 1 ppm by mass, the hue is unlikely to be deteriorated even when the absorbance is 0.05 or more in a wavelength range of from 250 nm to 800 nm, so that the monomer composition can be provided with a hue that is more suitable for a dental material.

In this manner, from the standpoint of inhibiting deterioration of the hue, the monomer composition of the disclosure is preferred when the tin-free catalyst (A) has an absorbance of 0.05 or more in a wavelength range of from 250 nm to 800 nm.

The absorbance of the tin-free catalyst (A) is measured using a UV-visible spectrophotometer ("V-650", manufactured by JASCO Corporation) in which a 0.01-mmol/L solution prepared by dissolving the tin-free catalyst (A) in tetrahydrofuran (containing no stabilizer) is placed in a 1 cm-long quartz cell.

The tin-free catalyst (A) contains a ligand, and this ligand may contain a group having at least one of a diketone structure or a double bond other than a carbonyl bond.

The double bond other than a carbonyl bond is likely to absorb light of a specific wavelength (e.g., visible light) to develop a color, or is likely to generate a hue through an addition reaction with a functional group different from a ketone in the process of producing the monomer composition. In the process of producing the monomer composition, the diketone structure is likely to generate a hue through an addition reaction with a functional group different from a ketone. The resulting addition-reacted group is likely to absorb light of a specific wavelength (e.g., visible light) and develop a color.

In the monomer composition of the disclosure, the content of an amide compound is less than 1 ppm by mass. Therefore, even when the ligand of the tin-free catalyst (A) contains a group having at least one of a diketone structure or a double bond other than a carbonyl bond, the hue of the monomer composition of the disclosure is suitable for a dental material.

The group having a double bond other than a carbonyl bond is, for example, an aromatic group, a thiocarbonyl group, a heteroaromatic group, or an alkenyl group. An aromatic group, a thiocarbonyl group, and a heteroaromatic group themselves are likely to exhibit a hue, and an alkenyl group is likely to generate a hue through an addition reaction. Examples of the aromatic group include a phenyl group, a naphthyl group, and a biphenyl group. Examples of a group having a thiocarbonyl group include a thiocarbamate group, a thiobenzamide group, a thioacetamide group, and a thiourea group. Examples of the heteroaromatic group include 3- to 6-membered rings having a nitrogen atom, a sulfur atom, or an oxygen atom, more specifically a furan group, a thiophene group, an oxazole group, a thiazole group, a pyridine group, a pyran group, and a thiopyran group. Examples of the alkenyl group include an allyl group, a vinyl group, a vinylidene group, and a vinylene group.

The group having a diketone structure may be a ligand having two ketone groups, and it is preferably a ligand containing a *β*-diketone. Examples of the ligand containing a *β*-diketone include acetylacetonate, 3-methyl acetylacetonate, 3-ethyl acetylacetonate, 3-phenyl acetylacetonate, trifluoroacetylacetonate, hexafluoroacetylacetonate, and diisobutylmethane.

The tin-free catalyst (A) preferably contains at least one of a compound represented by the following Formula (X) (hereinafter, referred to as "compound (X)"), a compound represented by the following Formula (Y) (hereinafter, referred to as "compound (Y)"), or a compound represented by the following Formula (Z) (hereinafter, referred to as "compound (Z)"), and more preferably contains the compound (X) or the compound (Y). When the tin-free catalyst (A) contains any of these compounds, it is likely to develop a hue; however, according to the monomer composition of the disclosure, such development of a hue can be inhibited.

The compound (X) is represented by the following Formula (X).

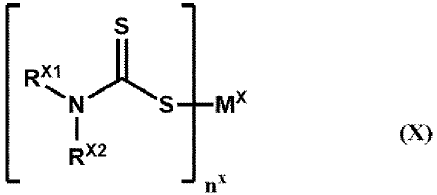

In Formula (X), each of R^{X1} and R^{X2} independently represents a monovalent hydrocarbon group; M^{X} represents a metal atom other than tin; n^{X} represents a valence of M^{X}; and plural R^{X1}s and R^{X2}s may be the same or different.

Examples of the monovalent hydrocarbon group represented by R^{X1} and R^{X2} include an alkyl group, an alkenyl group, an alkynyl group, an aryl group, an alkylaryl group, an alkenylaryl group, an alkynylaryl group, an arylalkyl group, an arylalkenyl group, and an arylalkynyl group.

It is preferred that each of R^{X1} and R^{X2} is independently an alkyl group or an aryl group.

Examples of the alkyl group include alkyl groups having from 1 to 6 carbon atoms, more specifically a methyl group, an ethyl group, a propyl group, and a butyl group.

Examples of the aryl group include a phenyl group.

M^{X} may be any metal atom other than tin as long as it has a catalytic activity, and M^{X} is preferably a specific metal atom, more preferably zinc, copper, indium, or platinum.

The term "specific metal atom" used herein refers to an atom of a metal element that belongs to at least one of Periods 4 to 6 and at least one of Groups 4 to 14. Specifically, the specific metal atom refers to titanium, vanadium, chromium, manganese, iron, cobalt, nickel, copper, zinc, gallium, germanium, zirconium, niobium, molybdenum, technetium, ruthenium, rhodium, palladium, silver, cadmium, indium, hafnium, tantalum, tungsten, rhenium, osmium, iridium, platinum, gold, mercury, thallium, or lead.

The tin-free catalyst (A) containing a compound (A) contains a compound represented by the below-described Formula (1).

Specific examples of the tin-free catalyst (A) containing a compound (A) include zinc dibutyl dithiocarbamate.

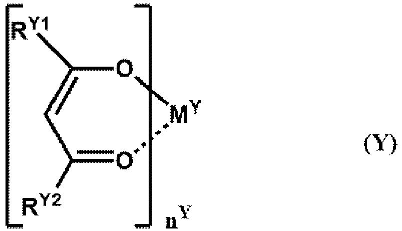

In Formula (Y), each of R^{Y1} and R^{Y2} independently represents a monovalent hydrocarbon group; M^{Y} represents a metal atom other than tin; n^{Y} represents a valence of M^{Y}; and plural R^{Y1}s and R^{Y2}s may be the same or different.

Examples of the "monovalent hydrocarbon group" represented by R^{Y1} and R^{Y2} include an alkyl group, an alkenyl group, an alkynyl group, an aryl group, an alkylaryl group, an alkenylaryl group, an alkynylaryl group, an arylalkyl group, an arylalkenyl group, and an arylalkynyl group.

It is preferred that each of R^{Y1} and R^{Y2} is independently an alkyl group or an aryl group.

Examples of the alkyl group include alkyl groups having from 1 to 6 carbon atoms, more specifically a methyl group, an ethyl group, a propyl group, and a butyl group.

Examples of the aryl group include a phenyl group.

M^{Y} may be any metal atom other than tin as long as it has a catalytic activity, and M^{Y} is preferably a specific metal atom, more preferably zinc, copper, indium, or platinum.

Specific examples of the tin-free catalyst (A) containing a compound (B) include indium acetylacetonate and platinum acetylacetonate.

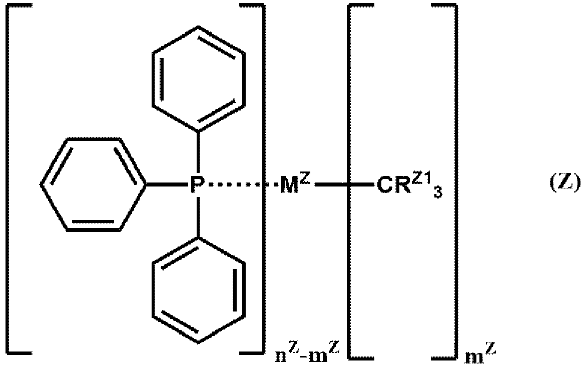

In Formula (Z), R^{Z1} represents a halogen atom; M^{Z} represents a metal atom other than tin; n^{Z} represents a valence of M^{Z}; and m^{Z} represents 1 or 0.

The halogen atom represented by R^{Z1} is preferably a fluorine atom, a chlorine atom, a bromine atom, or an iodine atom, more preferably a fluorine atom, a chlorine atom, or a bromine atom, still more preferably a fluorine atom or a chlorine atom, particularly preferably a fluorine atom.

M^{Z} may be any metal atom other than tin as long as it has a catalytic activity, and M^{Z} is preferably a specific metal atom, more preferably zinc, copper, indium, or platinum.

Specific examples of the tin-free catalyst (A) containing a compound (C) include tris(triphenylphosphine)(trifluoromethyl)copper.

The tin-free catalyst (A) preferably contains a metal atom other than tin. This metal atom other than tin is the specific metal atom, namely an atom of a metal element (excluding a tin atom) that belongs to at least one of Periods 4 to 6 and at least one of Groups 4 to 14. This allows the tin-free catalyst (A) to exert a catalytic activity in a synthesis reaction of the (meth)acrylate monomer (D) of the disclosure, even without containing a tin atom.

As the tin-free catalyst (A), a catalyst containing the above-described specific metal atom can be used, and examples thereof include zinc-based catalysts, titanium-based catalysts, zirconium-based catalysts, bismuth-based catalysts, iron-based catalysts, copper-based catalysts, cobalt-based catalysts, indium-based catalysts, and platinum-based catalysts.

For example, zinc-based catalysts contain a zinc atom. Titanium-based catalysts contain a titanium atom. Zirconium-based catalysts contain a zirconium atom. Bismuth-based catalysts contain a bismuth atom. Iron-based catalysts contain an iron atom. Copper-based catalysts contain a copper atom. Cobalt-based catalysts contain a cobalt atom. Indium-based catalysts contain an indium atom. Platinum-based catalysts contain a platinum atom.

The tin-free catalyst (A) preferably contains at least one selected from the group consisting of a zinc-based catalyst, a copper-based catalyst, an indium-based catalyst, and a platinum-based catalyst.

As the zinc-based catalyst, any known zinc-based catalyst can be used.

The zinc-based catalyst may be any catalyst containing at least one zinc atom, and it is preferably, for example, at least one of a sulfur-containing compound containing a sulfur atom or an oxygen-containing compound containing an oxygen atom.

The sulfur-containing compound containing a sulfur atom and the oxygen-containing compound containing an oxygen atom are each preferably at least one of a zinc atom-containing dithiocarbamate-based catalyst or a zinc compound containing a zinc atom and a ring structure.

The zinc atom-containing dithiocarbamate-based catalyst is preferably a zinc dithiocarbamate compound. The zinc compound containing a zinc atom and a ring structure is preferably a zinc compound that contains a zinc atom and an aromatic ring or a heterocycle, more preferably a zinc compound that contains a bisthiozinc skeleton (-S-Zn-S-) or a bisoxyzinc skeleton (-O-Zn-O-) and an aromatic ring or a heterocycle.

The tin-free catalyst (A) is preferably at least one of a compound represented by the following Formula (1) or a compound represented by the following Formula (2). The compound represented by the following Formula (2) may be a zinc complex. The tin-free catalyst (A) particularly preferably contains the compound represented by the following Formula (1).

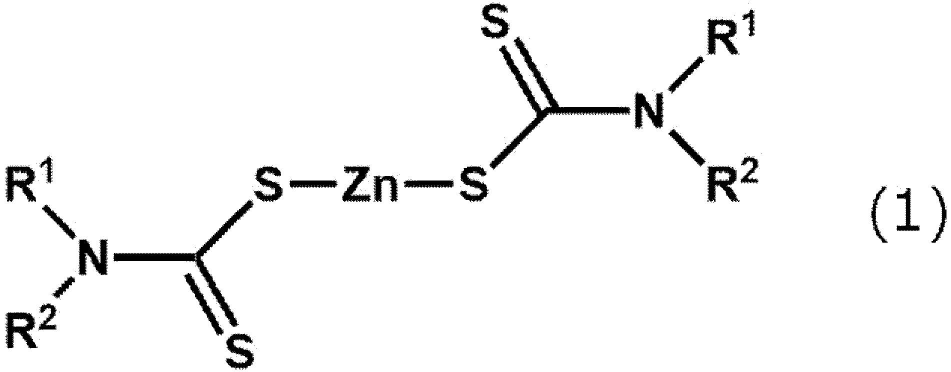

**R³-X¹-Zn-X²-R⁴** (2)

In Formula (1), each of two R¹s and two R²s independently represents a monovalent hydrocarbon group.

Examples of the monovalent hydrocarbon group represented by each of R¹s and R²s include an alkyl group, an alkenyl group, an alkynyl group, an aryl group, an alkylaryl group, an alkenylaryl group, an alkynylaryl group, an arylalkyl group, an arylalkenyl group, and an arylalkynyl group.

It is preferred that each of R¹s and R²s is independently an alkyl group or an aryl group.

Examples of the alkyl group include alkyl groups having from 1 to 6 carbon atoms, more specifically a methyl group, an ethyl group, a propyl group, and a butyl group.

Examples of the aryl group include a phenyl group.

Specific examples of the compound represented by Formula (1) include zinc dibutyl dithiocarbamate.

In Formula (2), each of X¹ and X² independently represents an oxygen atom or a sulfur atom. Each of R³ and R⁴ independently represents a monovalent organic group containing an aromatic ring or a heterocycle, or R³ and R⁴ are organic groups that are bound with each other to form a ring structure.

In R³ and R⁴, examples of the aromatic ring include a benzene ring, a naphthalene ring, an anthracene ring, and a phenanthrene ring, among which a benzene ring is preferred.

In R³ and R⁴, examples of the heterocycle include ring structures containing a nitrogen atom, a sulfur atom, an oxygen atom or the like, among which a heterocycle having aromaticity is preferred.

Specific examples of the heterocycle include a furan ring, a benzofuran ring, a pyrrole ring, an indole ring, an isoindole ring, a thiophene ring, a benzothiophene ring, an imidazole ring, a benzimidazole ring, a pyrazole ring, an indazole ring, an oxazole ring, a benzoxazole ring, a thiazole ring, a benzothiazole ring, a pyridine ring, a quinoline ring, a pyrazine ring, a quinoxaline ring, a pyrimidine ring, a pyridazine ring, and a triazine ring, among which a pyridine ring and a benzothiazole ring are preferred.

R³ and R⁴ may each contain one or more aromatic rings and one or more heterocycles, and may each contain plural aromatic rings or plural heterocycles. For example, R³ and R⁴ preferably each contain a benzene ring and a benzothiazole ring.

Examples of the ring structure formed by binding of R³ and R⁴ with each other include the above-exemplified aromatic rings and heterocycles, among which a benzene ring is preferred. When R³ and R⁴ are bound with each other, the ring structure preferably contains X¹, X², a zinc atom-containing ring, and an aromatic ring or a heterocycle. The ring structure is more preferably a fused ring sharing two carbon atoms.

Regarding R³ and R⁴, the monovalent organic group containing an aromatic ring or a heterocycle and the organic groups forming a ring structure may each independently contain a substituent. Examples of the substituent include alkyl groups such as those having from 1 to 6 carbon atoms, and aryl groups such as a phenyl group.

Specific examples of the compound represented by Formula (2) include compounds having the following structures.

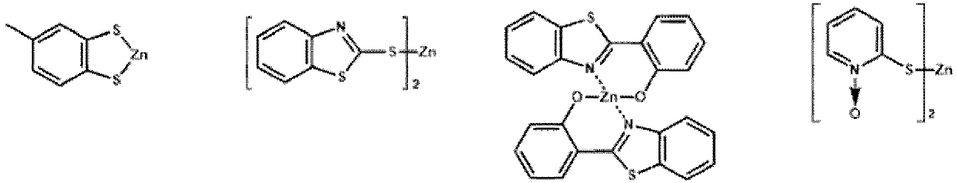

As the copper-based catalyst, any known copper-based catalyst can be used.

The copper-based catalyst may be any catalyst containing at least one copper atom, and may contain, for example, a copper atom and a ligand.

Examples of the ligand of the copper-based catalyst include acetylacetonate, tris(triphenylphosphine)(trifluoromethyl), and tetrakis(triphenylphosphine). Specific examples of the copper-based catalyst include tris(triphenylphosphine)(trifluoromethyl) copper.

As the indium-based catalyst, any known indium-based catalyst can be used.

The indium-based catalyst may be any catalyst containing at least one indium atom, and may contain, for example, an indium atom and a ligand. Examples of the ligand of the indium-based catalyst include the same ligands as those exemplified above for the ligand of the copper-based catalyst.

Specific examples of the indium-based catalyst include indium acetylacetonate.

As the platinum-based catalyst, any known platinum-based catalyst can be used.

The platinum-based catalyst may be any catalyst containing at least one platinum atom, and may contain, for example, a platinum atom and a ligand. Examples of the ligand of the platinum-based catalyst include the same ligands as those exemplified above for the ligand of the copper-based catalyst.

Specific examples of the platinum-based catalyst include platinum acetylacetonate.

In the monomer composition of the disclosure, the content of the tin-free catalyst (A) is preferably from 0.01% by mass to 1% by mass, more preferably from 0.03% by mass to 0.7% by mass, still more preferably from 0.05% by mass to 0.5% by mass, with respect to a total amount of the monomer composition.

In the monomer composition of the disclosure, the content of the zinc-based catalyst in particular may be 0.5% by mass or less, 0.3% by mass or less, or 0.1% by mass or less, with respect to a total amount of the monomer composition.

The monomer composition of the disclosure may or may not contain a catalyst that is the tin-free catalyst (A) but different from the zinc-based catalyst (hereinafter, also referred to as "other catalyst"). Examples of the other catalyst include catalysts containing a metal atom other than a tin atom and a zinc atom.

In the monomer composition of the disclosure, the content of the other catalyst may be 0.5% by mass or less, 0.3% by mass or less, or 0.1% by mass or less, with respect to a total amount of the monomer composition. A lower limit of the content of the other catalyst in the monomer composition of the disclosure is not particularly limited and, from the standpoint of reducing the use of a heavy metal such as tin, the lower limit may be 0% by mass.

### <(Meth)acrylate Monomer (D)>

The monomer composition of the disclosure contains a (meth)acrylate monomer (D).

In the monomer composition of the disclosure, the (meth)acrylate monomer (D) may be contained singly, or in combination of two or more kinds thereof.

The monomer composition of the disclosure can be used as, for example, a component in the below-described curable composition (specifically, as a monomer supply source for the below-described curable composition). The monomer composition of the disclosure itself can be used as a curable composition.

The (meth)acrylate monomer (D) is a reaction product of an iso(thio)cyanate compound (B) and an alcohol compound (C). The iso(thio)cyanate compound (B) contains an iso(thio)cyanate group. The alcohol compound (B) contains a hydroxyl group.

The (meth)acrylate monomer (D) preferably contains a urethane bond (i.e. at least one of a -NHC(=O)O- bond or a -NHC(=S)O- bond; the same applies hereinafter) that is formed by a reaction of the iso(thio)cyanate group contained in the iso(thio)cyanate compound (B) and the hydroxy group contained in the alcohol compound (C), and also contains a (meth)acryloyl group. The (meth)acryloyl group of the (meth)acrylate monomer (D) is preferably derived from at least one of the iso(thio)cyanate compound (B) or the alcohol compound (C). In other words, the (meth)acrylate monomer (D) is preferably urethane (meth)acrylate.

The (meth)acrylate monomer (D) preferably contains a thiourethane bond (i.e. at least one of a -NHC(=O)S- bond or a -NHC(=S)S- bond; the same applies hereinafter) that is formed by a reaction of the iso(thio)cyanate group contained in the below-described iso(thio)cyanate compound (b2) and a mercapto group contained in the below-described thiol compound (F), and also contains a (meth)acryloyl group. The (meth)acryloyl group of this (meth)acrylate monomer (D) is preferably derived from at least one of the iso(thio)cyanate compound (B) or the alcohol compound (C). In other words, the (meth)acrylate monomer (D) is preferably thiourethane (meth)acrylate.

This thiourethane (meth)acrylate preferably contains urethane bonds (i.e. a -NHC(=O)O- bond and a -NHC(=S)O- bond) that are formed by a reaction of the iso(thio)cyanate group contained in the iso(thio)cyanate compound (B) and the hydroxy group contained in the alcohol compound (C).

In the monomer composition of the disclosure, the content of the (meth)acrylate monomer (D) is preferably not less than 50% by mass, more preferably not less than 60% by mass, still more preferably not less than 80% by mass, particularly preferably not less than 90% by mass, with respect to a total amount of the monomer composition.

In the monomer composition of the disclosure, a total content of the (meth)acrylate monomer (D), the iso(thio)cyanate compound (B), and the alcohol compound (C) is preferably not less than 50% by mass, more preferably not less than 60% by mass, still more preferably not less than 80% by mass, particularly preferably not less than 90% by mass, with respect to a total amount of the monomer composition.

The iso(thio)cyanate compound (B) and the (meth)acrylate compound (C) that are contained in a raw material composition of the (meth)acrylate monomer (D) will now be described. Further, the thiol compound (F) that may be contained in the raw material composition of the (meth)acrylate monomer (D) will be described.

### (Iso(thio)cyanate Compound (B))

The raw material composition of the (meth)acrylate monomer (D) contains an iso(thio)cyanate compound (B).

The iso(thio)cyanate compound (B) contains an iso(thio)cyanate group.

In the monomer composition of the disclosure, the iso(thio)cyanate compound (B) containing an iso(thio)cyanate group may be contained singly, or in combination of two or more kinds thereof.

The number of iso(thio)cyanate groups in the iso(thio)cyanate compound (B) is preferably from 2 to 4.

It is preferred that at least one of the iso(thio)cyanate compound (B) or the alcohol compound (C) contains a (meth)acryloyl group. When the alcohol compound (C) contains a (meth)acryloyl group, the iso(thio)cyanate compound (B) may or may not contain a (meth)acryloyl group. When the alcohol compound (C) does not contain a (meth)acryloyl group, the iso(thio)cyanate compound (B) contains a (meth)acryloyl group.

The iso(thio)cyanate compound (B) preferably contains an aromatic ring.

In the disclosure, the iso(thio)cyanate compound (B) is, for example, an iso(thio)cyanate compound (b 1) or a reaction product (X).

The iso(thio)cyanate compound (b 1) is an iso(thio)cyanate compound itself that does not contain any thiourethane bond.

The reaction product (X) is a reaction product of an iso(thio)cyanate compound (b2) not containing a thiourethane bond, and a thiol compound (F) containing two or more mercapto groups. The details of the thiol compound (F) will be described below.

The iso(thio)cyanate compound (B) preferably contains the iso(thio)cyanate compound (b 1) or the reaction product (X).

Particularly, the iso(thio)cyanate compound (B) is preferably the reaction product (X). In other words, the (meth)acrylate monomer (D) is preferably a reaction product (Y) of the reaction product (X) and the alcohol compound (B). In this case, the (meth)acrylate monomer (D) contains a thiourethane bond formed by a reaction of the iso(thio)cyanate group contained in the reaction product (X) (i.e. iso(thio)cyanate group derived from the iso(thio)cyanate compound (b2)) and a mercapto group contained in the thiol compound (F), and also contains a (meth)acryloyl group.

Hereinafter, the iso(thio)cyanate compound (b1) and the iso(thio)cyanate compound (b2), which is a raw material of the reaction product (X), are collectively referred to as "iso(thio)cyanate compound (b)".

The iso(thio)cyanate compound (b) is preferably an isocyanate compound containing two or more (more preferably two or three, still more preferably two) isocyanate groups, more preferably at least one selected from the group consisting of hexamethylene diisocyanate, 2,2,4-trimethylhexamethylene diisocyanate, 2,4,4-trimethylhexamethylene diisocyanate, pentamethylene diisocyanate, m-xylylene diisocyanate, 1,3-tetramethylxylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, phenylene diisocyanate, 4,4'-diphenylmethane diisocyanate, 1,1-(bis(meth)acryloyloxymethyl)ethyl isocyanate, and 2- isocyanatoethyl (meth)acrylate.

The iso(thio)cyanate compound (b) (particularly the iso(thio)cyanate compound (b1) and the iso(thio)cyanate compound (b2)) more preferably contains at least one selected from the group consisting of m-xylylene diisocyanate, 1,3-tetramethylxylylene diisocyanate, tolylene diisocyanate, phenylene diisocyanate, and 4,4'-diphenylmethane diisocyanate.

As the iso(thio)cyanate compound (b), a thioisocyanate compound is, for example, an aliphatic polyisothiocyanate compound, an alicyclic polyisothiocyanate compound, an aromatic polyisothiocyanate compound, or a sulfur-containing heterocyclic polyisothiocyanate compound.

Examples of the aliphatic polyisothiocyanate compound include hexamethylene diisothiocyanate, lysine diisothiocyanatomethyl ester, lysine triisothiocyanate, m-xylylene diisothiocyanate, bis(isothiocyanatomethyl) sulfide, bis(isothiocyanatoethyl) sulfide, and bis(isothiocyanatoethyl) disulfide.

Examples of the alicyclic polyisothiocyanate compound include isophorone diisothiocyanate, bis(isothiocyanatomethyl)cyclohexane, dicyclohexylmethane diisothiocyanate, cyclohexane diisothiocyanate, methylcyclohexane diisothiocyanate, 2,5-bis(isothiocyanatomethyl)bicyclo-[2.2.1]-heptane, 2,6-bis(isothiocyanatomethyl)bicyclo-[2.2.1]-heptane, 3,8-bis(isothiocyanatomethyl)tricyclodecane, 3,9-bis(isothiocyanatomethyl)tricyclodecane, 4,8-bis(isothiocyanatomethyl)tricyclodecane, and 4,9-bis(isothiocyanatomethyl)tricyclodecane.

Examples of the aromatic polyisothiocyanate compound include tolylene diisothiocyanate, 4,4-diphenylmethane diisothiocyanate, and diphenyl disulfide-4,4-diisothiocyanate.

Examples of the sulfur-containing heterocyclic polyisothiocyanate compound include 2,5-diisothiocyanatothiophene, 2,5-bis(isothiocyanatomethyl)thiophene, 2,5-isothiocyanatotetrahydrothiophene, 2,5-bis(isothiocyanatomethyl)tetrahydrothiophene, 3,4-bis(isothiocyanatomethyl)tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane.

In the disclosure, when using a commercially available product as a raw material of the iso(thio)cyanate compound (B), it is preferred to use the iso(thio)cyanate compound (B) obtained by purifying the commercially available product by a known method. The commercially available product may contain an amide compound as a stabilizer. When such an amide compound-containing commercially available product is used directly as the iso(thio)cyanate compound (B) without purification, the amide compound contained in the commercially available product may cause the hue of the monomer composition to have a yellow tinge.

### (Alcohol Compound (C))

The raw material composition of the (meth)acrylate monomer (D) contains an alcohol compound (C).

The alcohol compound (C) contains an hydroxy group.

In the raw material composition of the (meth)acrylate monomer (D), the alcohol compound (C) may be contained singly, or in combination of two or more kinds thereof.

It is preferred that at least one of the iso(thio)cyanate compound (B) or the alcohol compound (C) contains a (meth)acryloyl group. When the iso(thio)cyanate compound (B) contains a (meth)acryloyl group, the alcohol compound (C) may or may not contain a (meth)acryloyl group. When the iso(thio)cyanate compound (B) does not contain a (meth)acryloyl group, the alcohol compound (C) contains a (meth)acryloyl group.

The alcohol compound (C) is preferably at least one selected from the group consisting of 2-hydroxyethyl (meth)acrylate, 2-hydroxypropyl (meth)acrylate, 2-hydroxybutyl (meth)acrylate, 2-hydroxy-3-phenoxypropyl (meth)acrylate, 4-hydroxybutyl (meth)acrylate, 1,4-cyclohexane dimethanol mono(meth)acrylate, ethylene glycol, and glycerin.

In the monomer composition of the disclosure, the molar ratio of the hydroxy group in the alcohol compound (C) with respect to the iso(thio)cyanate group in the iso(thio)cyanate compound (B) (i.e. molar ratio [hydroxy group/iso(thio)cyanate group]) is preferably from 0.3 to 2, more preferably from 0.5 to 1.5, still more preferably 0.8 to 1.2, yet still more preferably from 0.9 to 1.1.

In the monomer composition of the disclosure, a total content of the iso(thio)cyanate compound (B) and the alcohol compound (C) is preferably not less than 90% by mass, more preferably not less than 95% by mass, with respect to a total amount of the monomer composition.

An upper limit of the total content of the iso(thio)cyanate compound (B) and the alcohol compound (C) is determined as appropriate in accordance with the content of the tin-free catalyst (A). The upper limit of the total content of the iso(thio)cyanate compound (B) and the alcohol compound (C) is, for example, 99.9% by mass, 99.7% by mass, or 99.5% by mass.

### (Thiol Compound (F))

The raw material composition of the (meth)acrylate monomer (D) may also contain a thiol compound (F). By this, the (meth)acrylate monomer (D) is obtained as, for example, a reaction product (Y) of a reaction product (X), which is obtained by reacting the iso(thio)cyanate compound (b2) and the thiol compound (F), and the alcohol compound (C).

In the raw material composition of the (meth)acrylate monomer (D), the thiol compound (F) may be contained singly, or in combination of two or more kinds thereof.

The number of mercapto groups in the thiol compound (F) is preferably 2 or more, more preferably 3 or more, still more preferably from 3 to 6, particularly preferably from 3 to 5, further preferably 3 or 4.

With regard to the thiol compound (F), reference can be made as appropriate to, for example, the paragraphs [0045] to [0087] of WO 2019/107323.

The thiol compound (F) is preferably at least one selected from the group consisting of 4-mercaptomethyl-1,8-dimercapto-3,6-dithiaoctane, 4,8-dimercaptomethyl-1, 11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, pentaerythritol tetrakis(2-mercaptoacetate), pentaerythritol tetrakis(3-mercaptopropionate), 2,5-dimercaptomethyl-1,4-dithiane, bis(mercaptoethyl)sulfide, and diethylene glycol bis(mercaptopropionate).

The thiol compound (F) is more preferably at least one selected from the group consisting of 4-mercaptomethyl-1,8-dimercapto-3,6-dithiaoctane, 4,8-dimercaptomethyl-1, 11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, pentaerythritol tetrakis(2-mercaptoacetate), and pentaerythritol tetrakis(3-mercaptopropionate).

In the monomer composition of the disclosure, the molar ratio of the hydroxy group in the alcohol compound (C) and the mercapto group of the thiol compound (F) with respect to the iso(thio)cyanate group in the iso(thio)cyanate compound (b2) that is a raw material of the reaction product (X) (i.e. molar ratio [(hydroxy group + mercapto group)/iso(thio)cyanate group]) is preferably from 0.3 to 2, more preferably from 0.5 to 1.5, still more preferably 0.8 to 1.2, yet still more preferably from 0.9 to 1.1.

In the monomer composition of the disclosure, a total content of the iso(thio)cyanate compound (b2) that is a raw material of the reaction product (X), the alcohol compound (C), and the thiol compound (F) is preferably not less than 90% by mass, more preferably not less than 95% by mass, with respect to a total amount of the monomer composition.

An upper limit of the total content of the iso(thio)cyanate compound (b2), the alcohol compound (C), and the thiol compound (F) is determined as appropriate in accordance with the content of the tin-free catalyst (A). The upper limit of the total content of the iso(thio)cyanate compound (b2) that is a raw material of the reaction product (X), the alcohol compound (C), and the thiol compound (F) is, for example, 99.9% by mass, 99.7% by mass, or 99.5% by mass.

### (Stabilizer (E))

The raw material composition of the (meth)acrylate monomer (D) may also contain a stabilizer (E).

In the raw material composition of the (meth)acrylate monomer (D), the stabilizer (E) may be contained singly, or in combination of two or more kinds thereof.

The stabilizer (E) is at least one of a phosphite compound or a phenolic compound. In other words, the stabilizer (E) may be a phosphite compound, a phenolic compound, or a combination of a phosphite compound and a phenolic compound.

Examples of the phosphite compound include triphenyl phosphite, trisnonyl phenyl phosphite, tricresyl phosphite, diphenyl mono(2-ethylhexyl) phosphite, diphenyl monodecyl phosphite, diphenyl mono(tridecyl) phosphite, and tris(2,4-di-tert-butylphenyl) phosphite.

Examples of the phenolic compound include phenol, m-cresol, 2,5-tert-amylhydroquinone, and 2,5-di-tert-butylhydroquinone.

The content of the stabilizer (E) is preferably from 10 ppm by mass to 10,000 ppm by mass, more preferably from 50 ppm by mass to 5,000 ppm by mass, still more preferably from 100 ppm by mass to 2,500 ppm by mass, yet still more preferably from 200 ppm by mass to 1,000 ppm by mass, with respect to a total amount of the monomer composition.

The monomer composition of the disclosure may also contain, as an unreacted raw material, at least one of the iso(thio)cyanate compound (B) or the alcohol compound (C), in addition to the (meth)acrylate monomer (D) (i.e. reaction product).

### <Polymerization Inhibitor (G)>

The monomer composition of the disclosure may also contain a polymerization inhibitor (G).

When the monomer composition of the disclosure contains the polymerization inhibitor (G), the polymerization inhibitor (G) may be of a single kind, or a combination of two or more kinds.

The polymerization inhibitor (G) is not particularly limited, and examples thereof include dibutylhydroxytoluene (BHT), hydroquinone (HQ), hydroquinone monomethyl ether (MEHQ), and phenothiazine (PTZ).

The content of the polymerization inhibitor (G) is preferably from 0.001% by mass to 0.5% by mass, more preferably from 0.002% by mass to 0.3% by mass, still more preferably from 0.005% by mass to 0.3% by mass, yet still more preferably from 0.01% by mass to 0.2% by mass, with respect to a total amount of the monomer composition.

### <Other Monomer>

The monomer composition of the disclosure may contain at least one other monomer in addition to the (meth)acrylate monomer (D) as well as the iso(thio)cyanate compound (B) and the alcohol compound (C) that are raw materials thereof.

The other monomer is, for example, a (meth)acrylate other than the (meth)acrylate monomer (D), the iso(thio)cyanate compound (B), and the alcohol compound (C) (hereinafter, also referred to as "(meth)acrylate compound (H)").

Examples of the (meth)acrylate compound (H) include neopentyl di(meth)acrylate, 1,4-butanediol di(meth)acrylate, 1,6-hexanediol di(meth)acrylate, 1,8-octanediol di(meth)acrylate, 1,9-nonanediol di(meth)acrylate, 1,10-decanediol di(meth)acrylate, tricyclodecane dimethanol di(meth)acrylate, ethylene glycol di(meth)acrylate, diethylene glycol di(meth)acrylate, triethylene glycol di(meth)acrylate, tetraethylene glycol di(meth)acrylate, polyethylene glycol di(meth)acrylate, tripropylene glycol di(meth)acrylate, tetrapropylene glycol di(meth)acrylate, polypropylene glycol di(meth)acrylate, 2,2-bis[4-(3-(meth)acryloyloxy-2-hydroxypropoxy)phenyl]propane, ethylene oxide-modified bisphenol A di(meth)acrylate, and propylene oxide-modified bisphenol A di(meth)acrylate.

When the monomer composition of the disclosure contains other monomer (e.g., (meth)acrylate compound (E)), the content of the other monomer is preferably from 1% by mass to 70% by mass, more preferably from 5% by mass to 50% by mass, still more preferably from 10% by mass to 40% by mass, yet still more preferably from 20% by mass to 40% by mass, with respect to a total content of the iso(thio)cyanate compound (B), the alcohol compound (C), the (meth)acrylate monomer (D), and the other monomer (excluding a compound not containing a (meth)acryloyl group).

### <Other Components>

The monomer composition of the disclosure may also contain other components in addition to the above-described components.

Examples of the other components that may be contained in the monomer composition of the disclosure include the components contained in the below-described curable composition.

### [Method of Producing Monomer Composition]

The method of producing a monomer composition according to the disclosure is a method of producing a monomer composition containing a tin-free catalyst (A) and a (meth)acrylate monomer (D) that is a reaction product of an iso(thio)cyanate compound (B) and an alcohol compound (C). This method includes tmixing the tin-free catalyst (A), the iso(thio)cyanate compound (B), and the alcohol compound (C) (hereinafter, also referred to as "mixing step"), whrein a content of an amide compound containing at least one of a carboxylic acid amide group or a sulfonamide group is less than 1 ppm by mass with respect to a total amount of the monomer composition.

The method of producing a monomer composition according to the disclosure may also include other steps if necessary.

In the mixing step, by mixing the components of the tin-free catalyst (A), the iso(thio)cyanate compound (B), and the alcohol compound (C), a reaction of the iso(thio)cyanate compound (B) and the alcohol compound (C) is allowed to proceed, and the (meth)acrylate monomer (D) is generated as a reaction product. By this, the (meth)acrylate monomer (D) can be produced using a catalyst alternative to a Sn-based catalyst, and it is made possible to produce a cured product having a hue suitable for a dental material.

In the mixing step, the tin-free catalyst (A), an iso(thio)cyanate compound (b2) not containing a thiourethane bond, the alcohol compound (C), and a thiol compound (F) containing two or more mercapto groups may be mixed as well.

In the mixing step, by mixing the components of the tin-free catalyst (A), the iso(thio)cyanate compound (b2), the alcohol compound (C), and the thiol compound (F), a reaction of the iso(thio)cyanate compound (b2), the alcohol compound (C), and the thiol compound (F) is allowed to proceed, and the (meth)acrylate monomer (D) is generated as a reaction product.

In the mixing step, after mixing the tin-free catalyst (A), the iso(thio)cyanate compound (b2) not containing a thiourethane bond, and the thiol compound (F) containing two or more mercapto groups, the alcohol compound (C) may be added to and mixed with the resultant. In other words, in the mixing step, the iso(thio)cyanate compound (b2) and the thiol compound (F) may be reacted by mixing to generate a reaction product (X), and this reaction product (X) is subsequently reacted with the alcohol compound (C) to generate the (meth)acrylate monomer (D) as a reaction product (Y).

By this, the (meth)acrylate monomer (D) can be produced using a catalyst alternative to a Sn-based catalyst, and it is made possible to produce a cured product having a hue suitable for a dental material.

In the mixing step, an aspect of mixing the components is not particularly limited.

As the aspect of mixing the components, preferably, the tin-free catalyst (A) is mixed with one of the iso(thio)cyanate compound (B) and the alcohol compound (C) to prepare a composition first, and the other of the iso(thio)cyanate compound (B) and the alcohol compound (C) is subsequently added to and mixed with the thus obtained composition. In this case, the other of the iso(thio)cyanate compound (B) and the alcohol compound (C) may be slowly mixed by dropwise addition or the like.

As the aspect of mixing the components, more preferably, one of the iso(thio)cyanate compound (B) and the alcohol compound (C) contains a (meth)acryloyl group while the other does not contain a (meth)acryloyl group, and the components are mixed in the following manner. Specifically, the tin-free catalyst (A) is mixed with a compound that is one of the iso(thio)cyanate compound (B) and the alcohol compound (C) and does not contain a (meth)acryloyl group to prepare a composition, and a compound that is the other of the iso(thio)cyanate compound (B) and the alcohol compound (C) and does not contain a (meth)acryloyl group is subsequently added to and mixed with the thus obtained composition. This tends to allow the reaction of the iso(thio)cyanate compound (B) and the alcohol compound (C) to proceed in a preferred manner while inhibiting a polymerization reaction of the (meth)acryloyl group.

In the mixing step, the reaction temperature of the above-described reaction is preferably from 40°C to 90°C, more preferably from 50°C to 90°C, still more preferably from 60°C to 90°C.

In the mixing step, from the standpoint of allowing the reaction of the iso(thio)cyanate compound (B) and the alcohol compound (C) to proceed further, the reaction time of the above-described reaction is preferably 1 hour or longer, more preferably 2 hours or longer, still more preferably 3 hours or longer.

From the standpoint of further inhibiting, for example, decomposition, polymerization, and the like of the (meth)acrylate monomer (D) as well as a side reaction of iso(thio)cyanate compounds (B), the reaction time is preferably 30 hours or shorter, more preferably 25 hours or shorter, still more preferably 20 hours or shorter.

A preferred range of the amount of the tin-free catalyst (A) to be used in the mixing step is the same as the preferred range of the content of the tin-free catalyst (A) in the monomer composition of the disclosure.

Preferred ranges of the molar ratio [hydroxy group/iso(thio)cyanate group] and the total amount of the iso(thio)cyanate compound (B) and the alcohol compound (C) to be used in the mixing step are the same as those of the molar ratio [hydroxy group/iso(thio)cyanate group] and the total content of the iso(thio)cyanate compound (B) and the alcohol compound (C) in the monomer composition of the disclosure.

In the mixing step, not only the components of the tin-free catalyst (A), the iso(thio)cyanate compound (B), and the alcohol compound (C), but also other components may be added and mixed.

As other component, a polymerization inhibitor (G) is preferred. With regard to this polymerization inhibitor (G), reference can be made as appropriate to the above section of "Monomer Composition".

In the mixing step, when the components are mixed with an addition of the polymerization inhibitor (G), polymerization of the alcohol compound (C) can be further inhibited, so that the reaction of the iso(thio)cyanate compound (B) and the alcohol compound (C) is allowed to proceed more effectively.

The amount of the polymerization inhibitor (G) to be used may be from 0.001% by mass to 0.5% by mass, from 0.002% by mass to 0.3% by mass, or from 0.005% by mass to 0.3% by mass, with respect to a total amount of the tin-free catalyst (A), the iso(thio)cyanate compound (B), and the alcohol compound (C) that are used.

### [Curable Composition]

The curable composition of the disclosure contains the above-described monomer composition of the disclosure.

In other words, the curable composition of the disclosure contains the components contained in the above-described monomer composition of the disclosure.

The curable composition of the disclosure may consist of the above-described monomer composition of the disclosure (i.e. the monomer composition of the disclosure itself).

The curable composition of the disclosure is suitably used for the production of a cured product (e.g., the below-described molded body). The term "cured product" used herein means a product obtained by curing the curable composition of the disclosure.

Curing of the curable composition of the disclosure is realized by polymerization of the monomers contained in the curable composition.

Examples of a method of curing the curable composition of the disclosure include a method of polymerizing the monomers contained in the curable composition at normal temperature, a method of thermally polymerizing the monomers contained in the curable composition, and a method of photopolymerizing the monomers contained in the curable composition.

When the curable composition of the disclosure is made into a cured product, the cured product has a hue suitable for a dental material (e.g., without a yellow tinge).

It is believed that the above-described (meth)acrylate monomer (D) (i.e. the (meth)acrylate monomer (D) containing a urethane bond) as a monomer contributes to this effect.

The content of the monomer composition in the curable composition of the disclosure is preferably not less than 10% by mass, more preferably not less than 20% by mass, still more preferably not less than 30% by mass, with respect to a total amount of the curable composition.

The content of the monomer composition in the curable composition of the disclosure may be, for example, 100% by mass, 80% by mass or less, 60% by mass or less, or 50% by mass or less, with respect to a total amount of the curable composition.

### <Polymerization Initiator>

The curable composition of the disclosure preferably contains a polymerization initiator.

When the curable composition of the disclosure contains a polymerization initiator, the polymerization initiator may be of a single kind, or a combination of two or more kinds.

When the curable composition of the disclosure contains a polymerization initiator, polymerization of the monomers (i.e. the (meth)acrylate monomer (D) and other monomers that are contained if necessary; the same applies hereinafter) can be further facilitated in the process of curing the curable composition.

In the case of performing normal-temperature polymerization as the polymerization of the monomers, the polymerization initiator is preferably, for example, a redox-type polymerization initiator that is a combination of an oxidizing agent and a reducing agent.

In the case of using such a redox-type polymerization initiator, for example, the oxidizing agent and the reducing agent in the form of being separately packaged are prepared, and these agents may be mixed immediately before the use.

The oxidizing agent is not particularly limited and may be, for example, an organic peroxide. Examples of the organic peroxide include diacyl peroxides (e.g., benzoyl peroxide), peroxyesters (e.g., t-butyl peroxybenzoate), dialkyl peroxides (e.g., dicumyl peroxide), peroxyketals (e.g., 1,1-bis(t-butylperoxy)-3,3,5-trimethylcyclohexane), ketone peroxides (e.g., methyl ethyl ketone peroxide), and hydroperoxides (e.g., t-butyl hydroperoxide).

The reducing agent is not particularly limited and, usually, a tertiary amine (e.g., *N*,*N*-dimethylaniline) is used as the reducing agent.

In addition to these organic peroxide/amine-based polymerization initiators, examples of the redox-type polymerization initiator include cumene hydroperoxide/thiourea-based, ascorbic acid/Cu²⁺ salt-based, and organic peroxide/amine/sulfinic acid (or salt thereof)-based polymerization initiators.

As the polymerization initiator, tributyl borane, organic sulfinic acid, and the like can be preferably used as well.

In the case of performing thermal polymerization by heating as the polymerization of the monomers, the polymerization initiator is preferably, for example, a peroxide or an azo compound.

The peroxide is not particularly limited, and examples thereof include benzoyl peroxide, t-butyl hydroperoxide, and cumene hydroperoxide.

The azo compound is not particularly limited, and examples thereof include azobisisobutyronitrile.

In the case of performing photopolymerization by visible light irradiation as the polymerization of the monomers, the polymerization initiator (hereinafter, also referred to as "photopolymerization initiator") is preferably a redox-type initiator, such as *α*-diketone/tertiary amine, *α*-diketone/aldehyde, or α-diketone/mercaptan.

The photopolymerization initiator is not particularly limited and may be, for example, *α*-diketone/reducing agent, ketal/reducing agent, or thioxanthone/reducing agent.

Examples of the *α*-diketone include camphorquinone.

Examples of the ketal include benzyl dimethyl ketal.

Examples of the thioxanthone include 2-chlorothioxanthone.

Examples of the reducing agent include tertiary amines (e.g., Michler's ketone), aldehydes (e.g., citronellal), and thiol group-containing compounds (e.g., 2-mercaptobenzoxazole).

Further, polymerization initiators such as *α*-diketone/organic peroxide/reducing agent polymerization initiators obtained by adding an organic peroxide to any of the above-described redox-type initiators can also be preferably used as the photopolymerization initiator.

In the case of performing photopolymerization by UV irradiation, examples of the photopolymerization initiator include benzoin alkyl ethers, benzyl dimethyl ketals, and (bis)acylphosphine oxides.

Examples of the (bis)acylphosphine oxides include acylphosphine oxides (e.g., 2,4,6-trimethylbenzoyldiphenyl phosphine oxide), bis-acylphosphine oxides (e.g., bis-(2,6-dichlorobenzoyl)phenyl phosphine oxide).

These photopolymerization initiators of (bis)acylphosphine oxides may be used singly, or in combination with various reducing agents such as amines, aldehydes, mercaptans and sulfinates.

These photopolymerization initiators of (bis)acylphosphine oxides may also be used in combination with the above-described visible light photopolymerization initiators.

The polymerization initiator may be used referring to, for example, WO 2019/107323 and WO 2020/040141.

The content of the polymerization initiator is preferably from 0.01% by mass to 20% by mass, more preferably from 0.1% by mass to 5% by mass, with respect to a total amount of the monomers contained in the curable composition.

### <Filler>

The curable composition of the disclosure may also contain a filler.

When the curable composition of the disclosure contains a filler, the filler may be of a single kind, or a combination of two or more kinds.

When the curable composition of the disclosure contains a filler, the mechanical properties of a cured product obtained from the curable composition are further improved.

Fillers are broadly classified into organic fillers and inorganic fillers.

Examples of the organic fillers include fine powders of polymethyl methacrylate.

Examples of a material of the inorganic fillers include various glasses (e.g., glasses that contain silicon dioxide as a main component and, if necessary, an oxide of a heavy metal, boron, aluminum, or the like), various ceramics, diatomaceous earth, kaolin, clay minerals (e.g., montmorillonite), activated clay, synthetic zeolite, mica, calcium fluoride, ytterbium fluoride, calcium phosphate, barium sulfate, zirconium dioxide, titanium dioxide, and hydroxyapatite.

Specific examples of the inorganic fillers include barium borosilicate glass, strontium boroaluminosilicate glass, lanthanum glass, fluoroaluminosilicate glass, and boroaluminosilicate glass.

As the filler, for example, any of the fillers described in WO 2019/107323, WO 2020/040141, and the like may be used.

The content of the filler is preferably from 10 parts by mass to 2,000 parts by mass, more preferably from 50 parts by mass to 1,000 parts by mass, still more preferably from 100 parts by mass to 600 parts by mass, taking a total amount of the monomers contained in the curable composition as 100 parts by mass.

### <Other Components>

The curable composition of the disclosure may also contain other components in addition to the above-described components.

Examples of the other components include pigments, dyes, bactericides, disinfectants, stabilizers, and preservatives.

### <Preferred Use>

The use of the curable composition of the disclosure is not particularly limited.

The curable composition of the disclosure can be used as, for example, a coating material, a composition for forming a coating film, or a composition for a dental material.

The curable composition of the disclosure can form a cured product having a hue suitable for a dental material and is, therefore, particularly suitable as a composition for a dental material.

The term "composition for a dental material" used herein means a composition that can be used as is as a dental material, or a composition that can be used as a dental material in the form of a cured product of the composition for a dental material (e.g., the below-described molded body), or the cured product that is further processed.

Examples of the dental material include dental restoration materials, denture base resins, denture base lining materials, impression materials, dental luting materials (e.g., resin cements and resin-added glass ionomer cements), dental adhesives (e.g., orthodontic adhesives and cavity coating adhesives), dental fissure sealants, resin blocks for CAD (Computer Aided Design)/CAM (Computer Aided Manufacturing), temporary crowns, and artificial tooth materials.

Examples of the dental restoration materials include composite resins for crowns, carious cavity-filling composite resins, composite resins for abutment construction, and composite resins for filling restoration.

### <One Example of Production Method>

A method of producing the curable composition of the disclosure is not particularly limited.

One example of a method of producing the curable composition of the disclosure will now be described.

The method of producing the curable composition of the disclosure includes, for example: the step of preparing the monomer composition of the disclosure; and the step of mixing the monomer composition with other components (e.g., a polymerization initiator, a filler, etc).

The method of producing the curable composition of the disclosure may also include other steps if necessary.

### [Molded Body]

The molded body of the disclosure is a cured product of the above-described curable composition of the disclosure.

Therefore, the molded body of the disclosure has a hue suitable for a dental material.

The molded body is produced by, for example, molding the curable composition of the disclosure in a desired shape and subsequently curing the resultant.

Examples of a method of curing the curable composition of the disclosure are as described above.

### EXAMPLES

The disclosure will now be described more concretely by way of Examples; however, the disclosure is not limited to the below-described Examples.

Abbreviations of the compounds used in Examples of the disclosure are listed below.

### <Catalysts>

### (Tin-free Catalysts (A))

- ZDBDT: zinc dibutyldithiocarbamate
- ZACAC: zinc acetylacetonate
- InACAC: indium acetylacetonate
- PtACAC: platinum acetylacetonate
- PtTTPP: tetrakis(triphenylphosphine) platinum
- CuTTTF: tris(triphenyl phosphine)(trifluoromethyl) copper

### (Tin-based Catalyst)

- DBTDL: dibutyl tin dilaurate

### <Iso(thio)cyanate Compound (B)>

- XDI: m-xylylene diisocyanate

### <Alcohol Compound (C)>

- HPA: 2-hydroxypropyl acrylate

### <Stabilizers (E)>

### (Compounds Not Containing at Least One of Carboxylic Acid Amide Group or Sulfonamide Group)

- PhOH: phenol
- HCl: hydrochloric acid
- TPP: triphenyl phosphite

### (Compounds Containing at Least One of Carboxylic Acid Amide Group or Sulfonamide Group)

- PTSA: p-toluene sulfonamide
- BzA: benzamide

### <Thiol Compound (F)>

• THIOL: mixture of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane

### <Polymerization Inhibitor (G)>

- BHT: dibutylhydroxytoluene

### [Method of Measuring High-Performance Liquid Chromatography (HPLC)]

The HPLC chart spectrum of each (meth)acrylate obtained in Examples and Comparative Examples of the disclosure was measured using an HPLC apparatus "LC-20AT" manufactured by Shimadzu Corporation.

(Meth)acrylates obtained in Examples, Comparative Examples, and Reference Examples of the disclosure were each dissolved in CH₃CN and then measured in an eluent of CH₃CN/H₂O = 90/10.

### [Evaluation of Hue]

In Examples and Comparative Examples of the disclosure, the hue was defined by a difference (Δb*) between b* of each of the monomer compositions of Examples 3 to 16 and Comparative Examples 1 to 13 and b* of the monomer composition of Example 1 or 2. Specifically, the values of b* of the monomer compositions of Examples 3 and 4 and Comparative Examples 1 to 4, which did not contain the thiol compound (F) in a raw material of the respective monomer compositions, were compared against the b* of the monomer composition of Example 1 which did not contain the thiol compound (F) in a raw material of the monomer composition. The values of b* of the monomer compositions of Examples 5 and 6 and Comparative Examples 5 to 8, which contained the thiol compound (F) in a raw material of the respective monomer compositions, were compared against the b* of the monomer composition of Example 2 which contained the thiol compound (F) in a raw material of the monomer composition. The values of b* of the monomer compositions of Example 8 and Comparative Example 9, which contained the thiol compound (F) in a raw material of the respective monomer compositions, were compared against the b* of the monomer composition of Example 7 which contained the thiol compound (F) in a raw material of the monomer composition. The values of b* of the monomer compositions of Example 10 and Comparative Example 10, which contained the thiol compound (F) in a raw material of the respective monomer compositions, were compared against the b* of the monomer composition of Example 9 which contained the thiol compound (F) in a raw material of the monomer composition. The values of b* of the monomer compositions of Example 12 and Comparative Example 11, which contained the thiol compound (F) in a raw material of the respective monomer compositions, were compared against the b* of the monomer composition of Example 11 which contained the thiol compound (F) in a raw material of the monomer composition. The values of b* of the monomer compositions of Example 14 and Comparative Example 12, which contained the thiol compound (F) in a raw material of the respective monomer compositions, were compared against the b* of the monomer composition of Example 13 which contained the thiol compound (F) in a raw material of the monomer composition. The values of b* of the monomer compositions of Example 16 and Comparative Example 13, which contained the thiol compound (F) in a raw material of the respective monomer compositions, were compared against the b* of the monomer composition of Example 15 which contained the thiol compound (F) in a raw material of the monomer composition.

In Reference Examples of the disclosure, the hue was defined by a difference (Δb*) between b* of each of the monomer compositions of Reference Examples 3 and 4 and b* of the monomer composition of Reference Example 1 or 2. The b* of the monomer composition of Reference Example 3, which did not contain the thiol compound (F) in a raw material of the monomer composition, was compared against the b* of the monomer composition of Reference Example 1 which did not contain the thiol compound (F) in a raw material of the monomer composition. The b* of the monomer compositions of Reference Example 4, which contained the thiol compound (F) in a raw material of the monomer composition, was compared against the b* of the monomer composition of Reference Example 2 which contained the thiol compound (F) in a raw material of the monomer composition.

A Δb* of less than 0.3 was evaluated as "A", while a Δb* of 0.3 or more was evaluated as "B".

### (Method of Measuring b*)

The b* of each monomer composition of Examples and Comparative Examples of the disclosure was measured using a spectrocolorimeter (SD-3000, manufactured by Nippon Denshoku Industries Co., Ltd.). The temperature was controlled at 25°C.

### [Production Example: XDI]

An XDI product (manufactured by Tokyo Chemical Industry Co., Ltd., product code: "X0022", XDI purity: >98.0% (GC)) was charged to a distillation still equipped with a capillary tube connected to a nitrogen line. Vacuum distillation was performed in a pressure range of from 0.5 to 5 torr (from 66.7 Pa to 666.7 Pa) and in a temperature range of from 160°C to 240°C, and the resulting distillate was subsequently collected, whereby XDI was obtained from the XDI product.

### [Example 1]

In a thoroughly dried 100-mL four-necked flask equipped with a stirring blade and a thermometer, 41.97 parts by mass of XDI, 0.1 parts by mass of ZDBDT, and 0.05 parts by mass of BHT were added and dissolved to obtain a homogeneous solution, and this solution was heated to 80°C, after which 58.03 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount of HPA was added dropwise, reaction was allowed to proceed for 6 hours with reaction temperature being maintained at 90°C. In this process, the progress of the reaction was monitored by HPLC analysis to verify the endpoint of the reaction. The resulting product was discharged from the reactor, where by a monomer composition containing a urethane acrylate monomer (A-1) was obtained in an amount of 100 g.

### [Example 2]

In a thoroughly dried 100-mL four-necked flask equipped with a stirring blade and a thermometer, 42.70 parts by mass of XDI, 0.1 parts by mass of ZDBDT, 0.05 parts by mass of BHT, and 4.18 parts by mass of THIOL were added and dissolved to obtain a homogeneous solution, which was subsequently allowed to react at 80°C for 4 hours. This solution was heated to 90°C, and 53.12 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount was added dropwise, reaction was allowed to proceed for 6 hours with reaction temperature being maintained at 90°C. In this process, the progress of the reaction was monitored by HPLC analysis to verify the endpoint of the reaction. The resulting product was discharged from the reactor, where by a monomer composition containing a thiourethane acrylate monomer (A-2) was obtained in an amount of 100 g.

### [Example 3]

In a thoroughly dried 100-mL four-necked flask equipped with a stirring blade and a thermometer, 41.97 parts by mass of XDI, 0.1 parts by mass of ZDBDT, 0.05 parts by mass of BHT, 0.025 parts by mass of PhOH, and 0.025 parts by mass of HCl were added and dissolved to obtain a homogeneous solution, and this solution was heated to 80°C, after which 58.03 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount of HPA was added dropwise, reaction was allowed to proceed for 6 hours with reaction temperature being maintained at 90°C. In this process, the progress of the reaction was monitored by HPLC analysis to verify the endpoint of the reaction. The resulting product was discharged from the reactor, where by a monomer composition containing a urethane acrylate monomer (B-1) was obtained in an amount of 100 g.

### [Example 4]

A monomer composition containing a urethane acrylate monomer (B-2) was obtained in an amount of 100 g in the same manner as in Example 3, except that PhOH and HCl were changed to 0.05 parts by mass of TPP.

### [Example 5]

In a thoroughly dried 100-mL four-necked flask equipped with a stirring blade and a thermometer, 42.70 parts by mass of XDI, 0.1 parts by mass of ZDBDT, 0.05 parts by mass of BHT, 0.025 parts by mass of PhOH, 0.025 parts by mass of HCl, and 4.18 parts by mass of THIOL were added and dissolved to obtain a homogeneous solution, which was subsequently allowed to react at 80°C for 4 hours. This solution was heated to 90°C, and 53.12 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount was added dropwise, reaction was allowed to proceed for 6 hours with reaction temperature being maintained at 90°C. In this process, the progress of the reaction was monitored by HPLC analysis to verify the endpoint of the reaction. The resulting product was discharged from the reactor, where by a monomer composition containing a thiourethane acrylate monomer (B-3) was obtained in an amount of 100 g.

### [Example 6]

A monomer composition containing a thiourethane acrylate monomer (B-4) was obtained in an amount of 100 g in the same manner as in Example 5, except that PhOH and HCl were changed to 0.05 parts by mass of TPP.

### [Examples 7 to 16]

Monomer compositions were obtained each in an amount of 100 g in the same manner as in Example 5, except that the tin-free catalyst (A) and the stabilizer (E) were changed as shown in Table 1.

### [Comparative Example 1]

In a thoroughly dried 100-mL four-necked flask equipped with a stirring blade and a thermometer, 41.97 parts by mass of XDI, 0.1 parts by mass of ZDBDT, 0.05 parts by mass of BHT, and 0.05 parts by mass of PTSA were added and dissolved to obtain a homogeneous solution, and this solution was heated to 80°C, after which 58.03 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount of HPA was added dropwise, reaction was allowed to proceed for 6 hours with reaction temperature being maintained at 90°C. In this process, the progress of the reaction was monitored by HPLC analysis to verify the endpoint of the reaction. The resulting product was discharged from the reactor, where by a monomer composition containing a urethane acrylate monomer (C-1) was obtained in an amount of 100 g.

### [Comparative Example 2]

A monomer composition containing a urethane acrylate monomer (C-2) was obtained in an amount of 100 g in the same manner as in Comparative Example 1, except that PTSA was changed to BzA.

### [Comparative Example 3]

A monomer composition containing a urethane acrylate monomer (C-3) was obtained in an amount of 100 g in the same manner as in Comparative Example 1, except that the amount of PTSA was changed from 0.05 parts by mass to 0.005 parts by mass.

### [Comparative Example 4]

A monomer composition containing a urethane acrylate monomer (C-4) was obtained in an amount of 100 g in the same manner as in Comparative Example 2, except that the amount of BzA was changed from 0.05 parts by mass to 0.005 parts by mass.

### [Comparative Example 5]

In a thoroughly dried 100-mL four-necked flask equipped with a stirring blade and a thermometer, 42.70 parts by mass of XDI, 0.1 parts by mass of ZDBDT, 0.05 parts by mass of BHT, 0.05 parts by mass of PTSA, and 4.18 parts by mass of THIOL were added and dissolved to obtain a homogeneous solution, which was subsequently allowed to react at 80°C for 4 hours. This solution was heated to 90°C, and 53.12 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount was added dropwise, reaction was allowed to proceed for 6 hours with reaction temperature being maintained at 90°C. In this process, the progress of the reaction was monitored by HPLC analysis to verify the endpoint of the reaction. The resulting product was discharged from the reactor, where by a monomer composition containing a thiourethane acrylate monomer (C-5) was obtained in an amount of 100 g.

### [Comparative Example 6]

A monomer composition containing a thiourethane acrylate monomer (C-6) was obtained in an amount of 100 g in the same manner as in Comparative Example 5, except that PTSA was changed to BzA.

### [Comparative Example 7]

A monomer composition containing a thiourethane acrylate monomer (C-7) was obtained in an amount of 100 g in the same manner as in Comparative Example 5, except that the amount of PTSA was changed from 0.05 parts by mass to 0.005 parts by mass.

### [Comparative Example 8]

A monomer composition containing a thiourethane acrylate monomer (C-8) was obtained in an amount of 100 g in the same manner as in Comparative Example 6, except that the amount of BzA was changed from 0.05 parts by mass to 0.005 parts by mass.

### [Comparative Examples 9 to 13]

Monomer compositions were obtained each in an amount of 100 g in the same manner as in Comparative Example 5, except that the tin-free catalyst (A) and the stabilizer (E) were changed as shown in Table 1.

### [Reference Example 1]

In a thoroughly dried 100-mL four-necked flask equipped with a stirring blade and a thermometer, 41.97 parts by mass of XDI, 0.1 parts by mass of DBTDL, and 0.05 parts by mass of BHT were added and dissolved to obtain a homogeneous solution, and this solution was heated to 80°C, after which 58.03 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount of HPA was added dropwise, reaction was allowed to proceed for 4 hours with reaction temperature being maintained at 90°C. In this process, the progress of the reaction was monitored by HPLC analysis to verify the endpoint of the reaction. The resulting product was discharged from the reactor, where by a monomer composition containing a urethane acrylate monomer (D-1) was obtained in an amount of 100 g.

### [Reference Example 2]

In a thoroughly dried 100-mL four-necked flask equipped with a stirring blade and a thermometer, 42.70 parts by mass of XDI, 0.1 parts by mass of DBTDL, 0.05 parts by mass of BHT, and 4.18 parts by mass of THIOL were added and dissolved to obtain a homogeneous solution, which was subsequently allowed to react at 80°C for 2 hours. This solution was heated to 90°C, and 53.12 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount was added dropwise, reaction was allowed to proceed for 4 hours with reaction temperature being maintained at 90°C. In this process, the progress of the reaction was monitored by HPLC analysis to verify the endpoint of the reaction. The resulting product was discharged from the reactor, where by a monomer composition containing a thiourethane acrylate monomer (D-2) was obtained in an amount of 100 g.

### [Reference Example 3]

In a thoroughly dried 100-mL four-necked flask equipped with a stirring blade and a thermometer, 41.97 parts by mass of XDI, 0.1 parts by mass of DBTDL, 0.05 parts by mass of BHT, and 0.05 parts by mass of PTSA were added and dissolved to obtain a homogeneous solution, and this solution was heated to 80°C, after which 58.03 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount of HPA was added dropwise, reaction was allowed to proceed for 4 hours with reaction temperature being maintained at 90°C. In this process, the progress of the reaction was monitored by HPLC analysis to verify the endpoint of the reaction. The resulting product was discharged from the reactor, where by a monomer composition containing a urethane acrylate monomer (E-1) was obtained in an amount of 100 g.

### [Reference Example 4]

In a thoroughly dried 100-mL four-necked flask equipped with a stirring blade and a thermometer, 42.70 parts by mass of XDI, 0.1 parts by mass of DBTDL, 0.05 parts by mass of BHT, 0.05 parts by mass of PTSA, and 4.18 parts by mass of THIOL were added and dissolved to obtain a homogeneous solution, which was subsequently allowed to react at 80°C for 2 hours. This solution was heated to 90°C, and 53.12 parts by mass of HPA was further added thereto dropwise over a period of 1 hour. During the dropwise addition, since the internal temperature was increased by the reaction heat, the amount of added HPA was controlled such that the internal temperature was maintained to be 90°C or lower. After the whole amount was added dropwise, reaction was allowed to proceed for 4 hours with reaction temperature being maintained at 90°C. In this process, the progress of the reaction was monitored by HPLC analysis to verify the endpoint of the reaction. The resulting product was discharged from the reactor, where by a monomer composition containing a thiourethane acrylate monomer (E-2) was obtained in an amount of 100 g.

The results of measuring the b* and calculating the Δb* for the monomer compositions of Examples 1 to 16, Comparative Examples 1 to 13, and Reference Examples 1 to 4, and the results of evaluating the hue of the respective monomer compositions are shown in Tables 1 and 2.

**[Table 1]**

| | | Monomer synthesis | | | | | | | | Hue of monomer composition | | | |
|---|---|---|---|---|---|---|---|---|---|---|---|---|---|
| | | (A) | (B) | (C) | (E) | | | (F) | (G) | b* | b* (comparison subject) | Δb* | Evaluation |
| | | Tin-free catalyst | Iso(thio)cyanate compound | Alcohol compound | Stabilizer | | | Thiol compound | Polymerization inhibitor | | | | |
| | | | | | Amide-free compound | Amide-containing compound | | | | | | | |
| | | | | | | Type | Amount [ppm by mass] | | | | | | |
| Example | 1 | ZDBDT | XDI | HPA | - | - | 0 | - | BHT | 0.18 | - | - | - |
| | 2 | ZDBDT | XDI | HPA | - | - | 0 | THIOL | BHT | 0.21 | - | - | - |
| | 3 | ZDBDT | XDI | HPA | PhOH+HCl | - | 0 | - | BHT | 0.20 | 0.18 | 0.02 | A |
| | 4 | ZDBDT | XDI | HPA | TPP | - | 0 | - | BHT | 0.19 | 0.18 | 0.01 | A |
| | 5 | ZDBDT | XDI | HPA | PhOH+HCl | - | 0 | THIOL | BHT | 0.30 | 0.21 | 0.09 | A |
| | 6 | ZDBDT | XDI | HPA | TPP | - | 0 | THIOL | BHT | 0.24 | 0.21 | 0.03 | A |
| | 7 | ZACAC | XDI | HPA | - | - | 0 | THIOL | BHT | 0.22 | - | - | - |
| | 8 | ZACAC | XDI | HPA | PhOH+HCl | - | 0 | THIOL | BHT | 0.34 | 0.22 | 0.12 | A |
| | 9 | InACAC | XDI | HPA | - | - | 0 | THIOL | BHT | 0.24 | - | - | - |
| | 10 | InACAC | XDI | HPA | PhOH+HCl | - | 0 | THIOL | BHT | 0.42 | 0.24 | 0.18 | A |
| | 11 | PtACAC | XDI | HPA | - | - | 0 | THIOL | BHT | 0.22 | - | - | - |
| | 12 | PtACAC | XDI | HPA | PhOH+HCl | - | 0 | THIOL | BHT | 0.38 | 0.22 | 0.16 | A |
| | 13 | PtTTPP | XDI | HPA | - | - | 0 | THIOL | BHT | 0.24 | - | - | - |
| | 14 | PtTTPP | XDI | HPA | PhOH+HCl | - | 0 | THIOL | BHT | 0.41 | 0.24 | 0.17 | A |
| | 15 | CuTTTF | XDI | HPA | - | - | 0 | THIOL | BHT | 0.24 | - | - | - |
| | 16 | CuTTTF | XDI | HPA | PhOH+HCl | - | 0 | THIOL | BHT | 0.42 | 0.24 | 0.18 | A |
| Comparative Example | 1 | ZDBDT | XDI | HPA | - | PTSA | 500 | - | BHT | 0.72 | 0.18 | 0.54 | B |
| | 2 | ZDBDT | XDI | HPA | - | BzA | 500 | - | BHT | 0.70 | 0.18 | 0.52 | B |
| | 3 | ZDBDT | XDI | HPA | - | PTSA | 50 | - | BHT | 0.52 | 0.18 | 0.34 | B |
| | 4 | ZDBDT | XDI | HPA | - | BzA | 50 | - | BHT | 0.51 | 0.18 | 0.33 | B |
| | 5 | ZDBDT | XDI | HPA | - | PTSA | 500 | THIOL | BHT | 0.80 | 0.21 | 0.59 | B |
| | 6 | ZDBDT | XDI | HPA | - | BzA | 500 | THIOL | BHT | 0.72 | 0.21 | 0.51 | B |
| | 7 | ZDBDT | XDI | HPA | - | PTSA | 50 | THIOL | BHT | 0.55 | 0.21 | 0.34 | B |
| | 8 | ZDBDT | XDI | HPA | - | PTSA | 50 | THIOL | BHT | 0.54 | 0.21 | 0.33 | B |
| | 9 | ZACAC | XDI | HPA | - | PTSA | 50 | THIOL | BHT | 0.75 | 0.22 | 0.53 | B |
| | 10 | InACAC | XDI | HPA | - | PTSA | 50 | THIOL | BHT | 0.78 | 0.24 | 0.54 | B |
| | 11 | PtACAC | XDI | HPA | - | PTSA | 50 | THIOL | BHT | 0.73 | 0.22 | 0.51 | B |
| | 12 | PtTTPP | XDI | HPA | - | PTSA | 50 | THIOL | BHT | 0.75 | 0.24 | 0.51 | B |
| | 13 | CuTTTF | XDI | HPA | - | PTSA | 50 | THIOL | BHT | 0.77 | 0.24 | 0.53 | B |

**[Table 2]**

| | | Monomer synthesis | | | | | | | | | Hue of monomer composition | | | |
|---|---|---|---|---|---|---|---|---|---|---|---|---|---|---|
| | | (A) | Tin-based catalyst | (B) | (c) | (E) | | | (F) | (G) | b* | b* (comparison subject) | Δb* | Evaluation |
| | | Tin-free catalyst | | Iso(thio)cyanate compound | Alcohol compound | Stabilizer | | | Thiol compound | Polymerization inhibitor | | | | |
| | | | | | | Amide-free compound | Amide-containing compound | | | | | | | |
| | | | | | | | Type | Amount [ppm by mass] | | | | | | |
| Reference Example | 1 | - | DBTDL | XDI | HPA | - | - | 0 | - | BHT | 0.15 | - | - | - |
| | 2 | - | DBTDL | XDI | HPA | - | - | 0 | THIOL | BHT | 0.19 | - | - | - |
| | 3 | - | DBTDL | XDI | HPA | - | PTSA | 500 | - | BHT | 0.25 | 0.15 | 0.10 | A |
| | 4 | - | DBTDL | XDI | HPA | - | PTSA | 500 | THIOL | BHT | 0.30 | 0.19 | 0.11 | A |

In Tables 1 and 2, "amide-free compound" refers to a compound that does not contain at least one of a carboxylic acid amide group or a sulfonamide group. In Tables 1 and 2, "amide-containing compound" refers to a compound that contains at least one of a carboxylic acid amide group or a sulfonamide group.

UDMA (dimethacryloxyethyl-2,2,4-trimethylhexamethylene diurethane) and TEGDMA (triethylene glycol dimethacrylate), which are generally used as monomers for dental materials, have a b* value of 0.5 or less, and other monomers for dental materials are also required to have a hue of at least an equivalent level.

With regard to this point, the monomer compositions of Comparative Examples 1 to 4 contained the tin-free catalyst (A) and the urethane acrylate monomer (D) obtained as a reaction product of XDI (B) and HPA (C); however, the content of an amide-containing compound was 1 ppm by mass or more with respect to a total amount of the respective monomer compositions. The monomer compositions of Comparative Examples 5 to 13 contained the tin-free catalyst (A) and the thiourethane acrylate monomer (D) obtained as a reaction product of XDI (B), HPA (C), and THIOL (F); however, the content of an amide-containing compound was 1 ppm by mass or more with respect to a total amount of the respective monomer compositions. Therefore, the monomer compositions of Comparative Examples 1 to 13 all had a b* value of larger than 0.5. The hue of the monomer compositions of Comparative Examples 1 to 13 visually had a yellow tinge. In other words, it was found that the monomer compositions of Comparative Examples 1 to 13 did not have a hue suitable for a dental application.

On the other hand, the monomer compositions of Examples 1, 3, and 4 each contained the tin-free catalyst (A) and the urethane acrylate (D) obtained as a reaction product of XDI (B) and HPA (C), and the content of an amide-containing compound was less than 1 ppm by mass with respect to a total amount of the respective monomer compositions. The monomer compositions of Examples 2 and 5 to 16 each contained the tin-free catalyst (A) and the thiourethane acrylate (D) obtained as a reaction product of XDI (B), HPA (C), and THIOL (F), and the content of an amide-containing compound was less than 1 ppm by mass with respect to a total amount of the respective monomer compositions. Therefore, the monomer compositions of Examples 1 to 16 all had a b* value of 0.5 or less. The hue of the monomer compositions of Examples 1 to 16 did not visually have a yellow tinge. In other words, it was found that all of the monomer compositions of Examples 1 to 16 had a hue suitable for a dental material.

The monomer compositions of Reference Examples 1 to 4 contained a urethane acrylate monomer or a thiourethane acrylate monomer that was synthesized using a tin-based catalyst, DBTDL. Among these monomer compositions, those of Reference Examples 3 and 4 had a b* value of 0.5 or less, despite that the content of an amide-containing compound therein was 1 ppm by mass or more with respect to a total amount of the respective monomer compositions. The monomer compositions of Reference Examples 3 and 4 had hardly any difference Δb*, which is a difference in hue from that of the monomer composition of Reference Example 1 or 2 (in which the content of an amide-containing compound was less than 1 ppm by mass with respect to a total amount of the monomer composition).

According to these results, it is a problem unique to the use of a tin-free catalyst that a monomer composition synthesized in the process of synthesizing a urethane (meth)acrylate monomer or a thiourethane (meth)acrylate monomer sometimes has a hue with a yellow tinge. It was found that this problem can be solved by controlling the content of an amide-containing compound to be less than 1 ppm by mass.

The disclosure of Japanese Patent Application No. 2021-010402 filed on January 26, 2021 is hereby incorporated by reference in its entirety.

All the documents, patent applications, and technical standards that are described in the present specification are hereby incorporated by reference to the same extent as if each individual document, patent application, or technical standard is concretely and individually described to be incorporated by reference.

## Claims

1. A monomer composition, comprising:
a tin-free catalyst (A); and
a (meth)acrylate monomer (D) that is a reaction product of an iso(thio)cyanate compound (B) and an alcohol compound (C),
wherein a content of an amide compound comprising at least one of a carboxylic acid amide group or a sulfonamide group is less than 1 ppm by mass with respect to a total amount of the monomer composition.

2. The monomer composition according to claim 1, wherein:
the tin-free catalyst (A) comprises a ligand, and
the ligand comprises a group having at least one of a diketone structure or a double bond other than a carbonyl bond.

3. The monomer composition according to claim 1 or 2, wherein the tin-free catalyst (A) comprises at least one of a compound represented by the following Formula (X), a compound represented by the following Formula (Y), or a compound represented by the following Formula (Z): 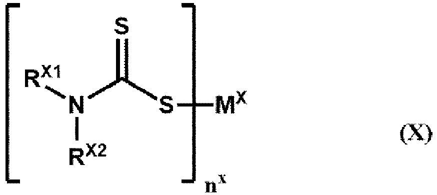 wherein, in Formula (X), each of R^{X1} and R^{X2} independently represents a monovalent hydrocarbon group; M^{X} represents a metal atom other than tin; n^{X} represents a valence of M^{X}; and plural R^{X1}s and R^{X2}s may be the same or different: 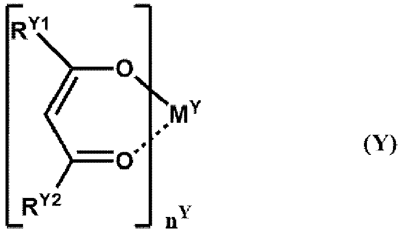 wherein, in Formula (Y), each of R^{Y1} and R^{Y2} independently represents a monovalent hydrocarbon group; M^{Y} represents a metal atom other than tin; n^{Y} represents a valence of M^{Y}; and plural R^{Y1}s and R^{Y2}s may be the same or different: 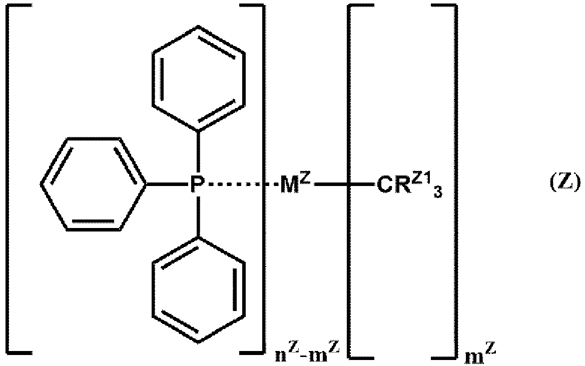 wherein, in Formula (Z), R^{Z1} represents a halogen atom; M^{Z} represents a metal atom other than tin; n^{Z} represents a valence of M^{Z}; and m^{Z} represents 1 or 0.

4. The monomer composition according to any one of claims 1 to 3, wherein:
the tin-free catalyst (A) comprises a metal atom other than tin, and
the metal atom other than tin is an atom of a metal element (excluding a tin atom) that belongs to at least one of Periods 4 to 6 and at least one of Groups 4 to 14.

5. The monomer composition according to any one of claims 1 to 4, wherein the tin-free catalyst (A) comprises at least one selected from the group consisting of a zinc-based catalyst, a copper-based catalyst, an indium-based catalyst, and a platinum-based catalyst.

6. The monomer composition according to any one of claims 1 to 5, wherein the iso(thio)cyanate compound (B) comprises an aromatic ring.

7. The monomer composition according to any one of claims 1 to 6, wherein the iso(thio)cyanate compound (B) comprises an iso(thio)cyanate compound (b1) not containing a thiourethane bond or, a reaction product (X) of a thiol compound (F) containing two or more mercapto groups and an iso(thio)cyanate compound (b2) not containing a thiourethane bond.

8. The monomer composition according to claim 7, wherein the iso(thio)cyanate compound (b1) and the iso(thio)cyanate compound (b2) each comprise at least one selected from the group consisting of m-xylylene diisocyanate, 1,3-tetramethylxylylene diisocyanate, tolylene diisocyanate, phenylene diisocyanate, and 4,4'-diphenylmethane diisocyanate.

9. The monomer composition according to any one of claims 1 to 8, wherein the alcohol compound (C) comprises at least one selected from the group consisting of 2-hydroxyethyl (meth)acrylate, 2-hydroxypropyl (meth)acrylate, 2-hydroxybutyl (meth)acrylate, 2-hydroxy-3-phenoxypropyl (meth)acrylate, 4-hydroxybutyl (meth)acrylate, 1,4-cyclohexane dimethanol mono(meth)acrylate, ethylene glycol, and glycerin.

10. The monomer composition according to any one of claims 1 to 9, comprising a stabilizer (E) which is at least one of a phosphite compound or a phenolic compound.

11. The monomer composition according to any one of claims 1 to 10, wherein the iso(thio)cyanate compound (B) is a reaction product of the iso(thio)cyanate compound (b2) not containing a thiourethane bond and the thiol compound (F) containing two or more mercapto groups.

12. A curable composition, comprising the monomer composition according to any one of claims 1 to 11.

13. The curable composition according to claim 12, further comprising a polymerization initiator.

14. The curable composition according to claim 12 or 13, which is a composition for a dental material.

15. A molded body, comprising a cured product of the curable composition according to any one of claims 12 to 14.

16. A method of producing a monomer composition comprising a tin-free catalyst (A) and a (meth)acrylate monomer (D) that is a reaction product of an iso(thio)cyanate compound (B) and an alcohol compound (C), the method comprising:
mixing the tin-free catalyst (A), the iso(thio)cyanate compound (B), and the alcohol compound (C),
wherein a content of an amide compound comprising at least one of a carboxylic acid amide group or a sulfonamide group is less than 1 ppm by mass with respect to a total amount of the monomer composition.
